(19) Europäisches Patentamt
European Patent Office
Office européen des brevets

(11) Publication number: 0 317 204 A2

(12) **EUROPEAN PATENT APPLICATION**

(21) Application number: 88310659.3

(22) Date of filing: 11.11.88

(51) Int. Cl.4: C07C 69/708 , C07D 239/26 , C07D 239/34 , C07D 213/30 , C07D 213/65 , C09K 19/12 , C09K 19/34

(30) Priority: 13.11.87 JP 287017/87

(43) Date of publication of application: 24.05.89 Bulletin 89/21

(84) Designated Contracting States: CH DE FR GB IT LI NL SE

(71) Applicant: Chisso Corporation
6-32, Nakanoshima 3-chome Kita-ku
Osaka-shi Osaka-fu(JP)

(72) Inventor: Ohno, Kouji
10-3, Otsutomo-cho Kanazawa-ku
Yokohama-shi Kanagawa-ken(JP)
Inventor: Inoue, Hiromichi
10-2, Otsutomo-cho Kanazawa-ku
Yokohama-shi Kanagawa-ken(JP)
Inventor: Saito, Shinichi
10-1, Otsutomo-cho Kanazawa-ku
Yokohama-shi Kanagawa-ken(JP)
Inventor: Miyazawa, Kazutoshi
12-14, Mutsuura 2-chome Kanazawa-ku
Yokohama-shi Kanagawa-ken(JP)
Inventor: Ushida, Makoto
12-16, Kannon 1-chome Kawasaki-ku
Kawasaki-shi Kanagawa-ken(JP)

(74) Representative: Lamb, John Baxter et al
MARKS & CLERK 57/60 Lincoln's Inn Fields
London WC2A 3LS(GB)

(54) **Alpha-aryloxypropionic acid esters.**

(57) Optical active compound, particularly useful as components of ferroelectric liquid crystal compositions, have the formula:

$$R^1 - A - O\overset{*}{\underset{}{C}}H(CH_3)\overset{O}{\overset{\|}{C}}OR^2 \qquad (I)$$

in which $R^1$ is a straight or branched alkyl or alkoxy group containing up to 18 carbon atoms, $R^2$ is a straight or branched alkyl group containing up to 12 carbon atoms, -A- is a group

EP 0 317 204 A2

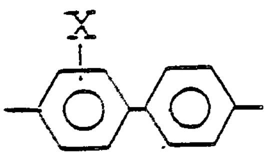 , 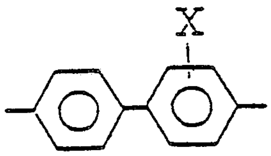 , 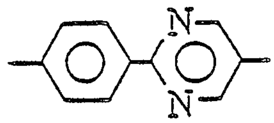 , 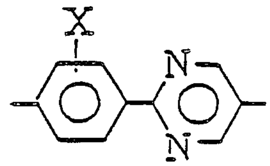 ,

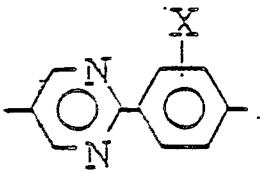 , 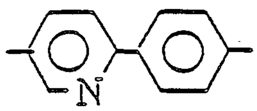 or 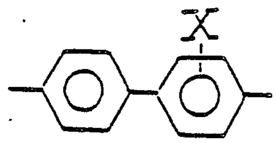

(wherein X is a fluorine or chlorine atom or a cyano group and * indicates an asymmetric carbon atom.

## Alpha-Aryloxypropionic acid esters

This invention relates to a novel liquid crystalline compound having an optically active group and useful in the fields of liquid crystal display elements and liquid crystal light switching elements, and a liquid crystal composition containing the same. More particularly, it relates to an optically active compound particularly useful as a component of ferroelectric liquid crystal compositions.

It is well known that the improtance of optically active substances in liquid crystal compositions used for electrooptic elements utilizing liquid crystal pahses i.e. liquid crystal display elements or liquid crystal light switching elements, consists in the usefulness thereof as a component constituting

i) cholesteric phase (chiral nematic phase) and

ii) chiral smectic phase.

It is also well known that cholesteric phase (Ch phase) may be composed of compounds exhibiting Ch phase by themselves as main components, but the pahse may also be composed by adding an optically active substance to nematic phase, and in the latter case, the added optically active substance itself is not always necessary to exhibit liquid crystal phase. It is also known that Ch phase is not only used for display elements utilizing cholestericnematic phase transition, but also Ch phase is used in place of neamtic phase in order to prevent occurrence of reverse domain in the so-called TN cell (Twisted Nematic cell). Further, as a component constituting cholesteric liquid crystal compositions in STN mode (a mode wherein the twist angle of liquid crystal has been about 180° -270°) as one of relatively novel display modes, the importance of optically active substances has been more and more increasing.

On the other hand, electro-optic elements using chiral smectic liquid crystals have recently been noted. The elements utilize ferroelectricity of liquid crystals and in the case of this novel display mode, there is a possibility of notably improving the response rate (Clark et al; Applied Phys. lett., 36,899 (1980)). This display mode is a method utilizing chiral smectic phases exhibiting ferroelectricity such as chiral smectic C phase (hereinafter abbreviated to SC*). Ferroelectricity-exhibiting phases are not only limited to SC* phase, but chiral smectic F, G, H, I phases and the like, too, have been known to exhibit ferroelectricity. When these ferroelectric liquid crystals are used for display elements, liquid crystal materials the ferroelectric liquid crystal properties of which are exhibited over a broad temperature range including room temperature have been desired. At present, however, no single compound satisfying such a requirement has been known. Thus, liquid crystal compositions prepared by combining some compounds to thereby satisfy such a requirement as much as possible have been used.

Chiral smectic liquid crystal compositions may be composed of compounds exhibiting chiral smectic phase by themselves, as main components, as in the case of Ch phase, but they may also be composed by adding an optically active substance to smectic phase, and in this case, too, the added optically active substance itself is not always necessary to exhibit liquid crystal phase.

The present inventors have made extensive research on various compounds in order to find optically active liquid crystalline compounds useful as a component of liquid crystal compositions as described above, and as a result have achieved the present invention. In addition, liquid crystalline compounds referred to herein include not only compounds the liquid crystal state of which can be observed by themselves, but also substances the liquid crystal state of which cannot be observed by themselves, but which have a chemical structure similar to that of the former and useful as an additive as described above.

## SUMMARY OF THE INVENTION

The present invention resides in;

(1) an optically active compound expressed by the formula

$$R^1-A-O\underset{*}{\overset{\overset{CH_3}{|}}{C}}H\overset{\overset{O}{\|}}{C}OR^2 \qquad (I)$$

wherein $R^1$ represents a linear or branched alkyl group or alkoxy group each of 1 to 18 carbon atoms, $R^2$ represents a linear or branched alkyl group of 1 to 12 carbon atoms, -A- represents

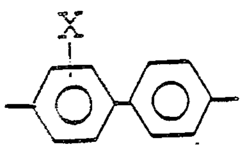 , 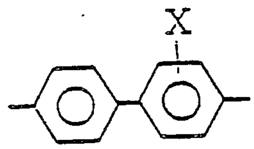 , 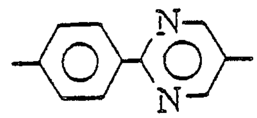 , 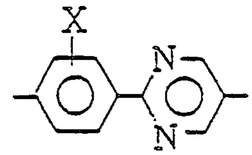 ,

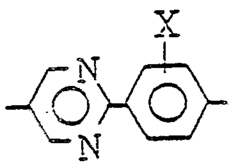 , 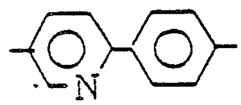 or 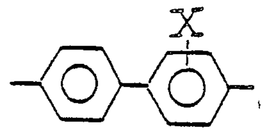

wherein X represents F, Cℓ or cyano group, and * indicates an asymmetric carbon atom ;

(2) a liquid crystal composition, particularly a chiral smectic liquid crystal composition, comprising at least two components at least one of which is a compound as set forth in item (1); and

(3) an electro-optic element comprising a liquid crystal composition as set forth in item (2).

DETAILED DESCRIPTION OF PREFERRED EMBODIMENTS

$R^1$ in the formula (I) is preferably a linear or branched alkyl or alkoxy group each of 4 to 15 carbon atoms, more preferably a linear or branched alkyl or alkoxy group each of 6 to 12 carbon atoms. $R^2$ is preferably a linear or branched alkyl group of 2 to 10 carbon atoms and in the case of a branched alkyl group, $R^2$ may be an optically active group.

The phase transition points of representative examples of the compound of the formula (I) of the present invention are shown in Table 1.

Table 1

| compound No. | In formula (I) R[1] | A | R[2] | m.p. (°C) | Note |
|---|---|---|---|---|---|
| 1 | $C_6H_{13}$ | | $C_2H_5$ | 17.3 | |
| 2 | $C_8H_{17}$ | | $C_2H_5$ | 6.3 | |
| 3 | $C_{10}H_{21}$ | 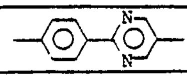 | $C_2H_5$ | 22.0 | |
| 4 | $C_7H_{15}O$ | | $C_2H_5$ | 72.0 | |
| 5 | $C_8H_{17}O$ | | $C_2H_5$ | 90.0 | |
| 6 | $C_9H_{19}O$ | | $C_2H_5$ | 74.6 | |
| 7 | $C_{12}H_{25}O$ | | $C_2H_5$ | 87.4 | |
| 8 | $C_6H_{13}$ | | $C_2H_5$ | oil at r.t. | |
| 9 | $C_7H_{15}$ | 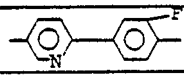 | $C_2H_5$ | oil at r.t. | |
| 10 | $C_8H_{17}$ | | $C_2H_5$ | oil at r.t. | |
| 11 | $C_7H_{15}$ | | $C_2H_5$ | oil at r.t. | |
| 12 | $C_8H_{17}$ | 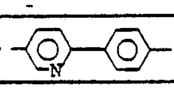 | $C_2H_5$ | oil at r.t. | Example 1 |
| 13 | $C_9H_{19}$ | | $C_2H_5$ | -5.8 | |
| 14 | $C_{10}H_{21}$ | | $C_2H_5$ | 10.7 | |
| 15 | $C_{12}H_{25}$ | 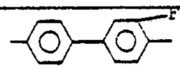 | $C_2H_5$ | 39.1 | |
| 16 | $C_{12}H_{25}$ | 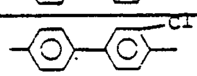 | $C_2H_5$ | -9.0 | |
| 17 | $C_4H_9$ | | $C_2H_5$ | 53.1 | |
| 18 | $C_5H_{11}$ | | $C_2H_5$ | oil at r.t. | |
| 19 | $C_8H_{17}$ | 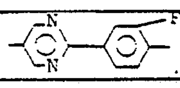 | $C_2H_5$ | 10.3 | |
| 20 | $C_8H_{17}$ | | $C_3H_7$ | oil at r.t. | |
| 21 | $C_8H_{17}$ | | $C_6H_{13}$ | oil at r.t. | |
| 22 | $C_8H_{17}$ | | $C_7H_{15}$ | oil at r.t. | |
| 23 | $C_9H_{19}$ | | $C_2H_5$ | 10.7 | |

When the liquid crystalline compound of the present invention is used as a component of ferroelectric liquid crystal compositions, it is possible to enlarge the spontaneous polarization value Ps of the compositions. Here, the importance of the Ps will be briefly described. In general, the response time $\tau$ of ferroelectric liquid crystal display elements is expressed by the formula (II)

$$\tau = \frac{\eta}{Ps \cdot E} \qquad (II)$$

wherein $\eta$ represents a viscosity and E represents an electric field intensity.

As apparent from the formula (II), in order to reduce the response time, the Ps may be increased or the viscosity may be reduced.

When the compound of the present invention is used as a component of ferroelectric liquid crystal compositions, it has a function of notably increasing the Ps of the ferroelectric liquid crystal compositions and as a result, it is possible to reduce the response time. As described later in Examples, when a compound of the formula (I) of the present invention is added in 20% by weight to a liquid crystal composition exhibiting an achiral smectic C phase and exhibiting no spontaneous polarization, a ferroelectric liquid crystal composition having a large spontaneous polarization value is obtained, and when this composition is used, a display element exhibiting a short response time of 120 $\mu$ sec at 25 °C is obtained (see Example 2). Further, when a compound of the formula (I) of the present invention is added to a liquid crystal composition exhibiting a chiral smectic C phase, but exhibiting a very small Ps, it is possible to raise the Ps up to a practical value. In short, the compound of the present invention is far superior as a component bearing the Ps of ferroelectric liquid crystal compositions.

Further, the compound of the formula (I) of the present invention has an optically active carbon atom; hence when it is added to nematic liquid crystals, it has a capability of inducing a twisted structure. Namely, nematic liquid crystals having a nematic structure i.e. chiral nematic liquid crystals do not form the so-called reverse domain (interference fringes) of TN display mode elements; hence the compound of the formula (I) is usable as an agent for preventing formation of the reverse domain.

The compound of the present invention may be prepared for example through the following route:

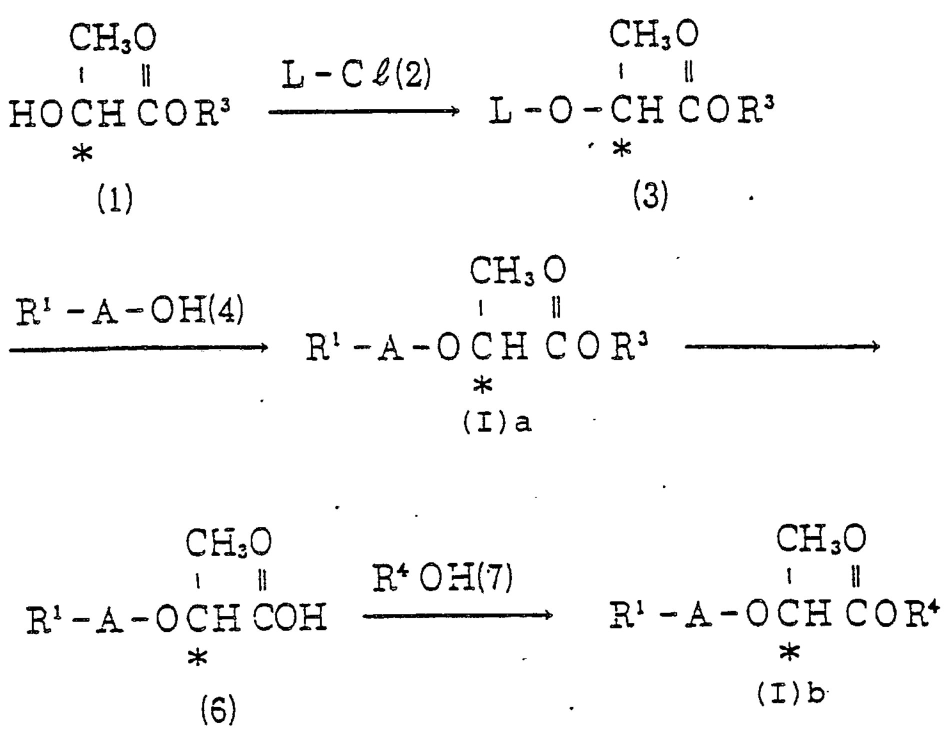


wherein $R^1$ and -A- are as defined above, L-represents a group such as methanesulfonyl, benzenesulfonyl, p-toluenesulfonyl, etc. and $R^3$ and $R^4$ each represent an alkyl group of 1 to 18 carbon atoms.

Namely, a sulfonyl chloride (2) such as methanesulfonyl chloride, p-toluenesulfonyl chloride or the like is reacted with an alkyl lactate (1), followed by reacting a phenol (4) with the resulting sulfonylated compound (3) to obtain a compound of the present invention (I)a. This compound (I)a is further hydrolyzed into a carboxylic acid (6), which is then esterified with an alcohol (7) to obtain a compound of the present invention having another alkyl group (I)b.

The compound and liquid crystal composition of the present invention will be described in more detail by way of Examples.

## Example 1

Preparation of R-2-[4-(5-octylpyridyl-2)phenoxy]propionic acid ethyl ester

(a compound of the formula (I) wherein $R^1$ represents octyl group, $R^2$ represents ethyl group and -A- represents

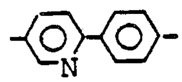 )

(Compound No. 12)

i) Preparation of (S)-2-(methanesulfonyloxy)propionic acid ethyl ester

(S)-ethyl lactate (118.0 g, 1 mol) and triethylamine (126.5 g, 1.25 mol) were dissolved in methylene chloride (500 mℓ), followed by agitating the solution under ice cooling, dropwise adding thereto methanesulfonyl chloride (126.0 g, 1.1 mol), agitating the mixture for 3 hours, adding cold water (1 ℓ), shaking the mixture, separating an organic layer,washing this organic layer with 6N-HCℓ aqueous solution, then with 2N-NaOH aqueous solution and further with water until the washing water became neutral, distilling off the solvent and distilling the residue under reduced pressure to obtain the objective (S)-2-(methanesulfonyloxy) ethyl propionate (168.0 g) in the form of colorless liquid (106~109 °C/5 mmHg, $\alpha_D^{20}$ - 65.7 (ℓ = 1, neat)).

ii) Preparation of R-2-[4-(5-octylpyridyl-2)phenoxy]propionic acid ethyl ester

4-(5-Octyl-pyridine-2-yl)phenol (10.0 g, 35 mmol) prepared in the same manner as described in a literature (A.I. Pavulchenko et al; editor L. Bata, Advance in Liquid Crystal Research and Applications, Pergmon Press, 1980, p. 1007) was dissolved in acetonitrile (120 mℓ), followed by adding anhydrous $K_2CO_3$ (4.9 g, 35 mmol) to the solution, dropwise adding to the mixture, (S)-2-(methanesulfonyloxy)ethyl propionate (6.5 g) obtained above in item i), keeping the mixture under refux for 14 hours, allowing the resulting material to cool down, adding water (200 mℓ) and toluene (100 mℓ), separating an organic layer, washing the layer with 6N-hydrochloric acid, then with 2N-NaOH aqueous solution and further with water until the washing water became neutral, distilling off the solvent and recrystallizing the residue from ethanol to obtain R-2-[4-(5-octylpyridyl-2)phenoxy]propinic acid ethyl ester (8.7 g). This product was oily at room temperature.

## Example 2

A composition A as an achiral substance having SC phase was prepared. The phase transition points of this composition A was as follows:

$$C \longrightarrow SC \xrightarrow{49.1°C} SA \xrightarrow{52.2°C} I$$

wherein I represents an isotropic phase.

To this composition A was added the compound of Example 1 of the present invention i.e.

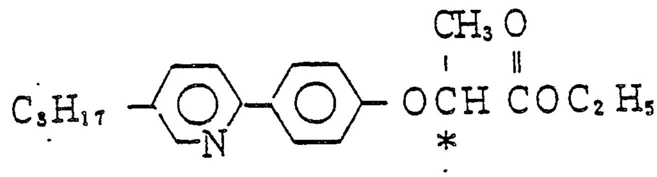

in 20% by weight. As a result, SC* phase exhibiting ferroelectricity at 30°C or lower appeared. The spontaneous polarization value at 25°C of this composition was 25 nC/cm² and its tilt angle was 20°. This composition was filled in a cell of 2 µm thick provided with transparent electrodes each obtained by coating PVA as an aligning agent and further rubbing the resulting surface to subject it to a parallel aligning treatment, followed by placing the cell between two crossed polarizers and impressing a square wave of wave height of 10 V. As a result, change in the intensity of transmitted light was observed. The response time as sought from the change in the intensity of transmitted light at that time was 120 µsec at 25°C.

Example 3

A nematic liquid crystal composition consisting of

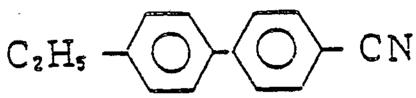

20 wt. %

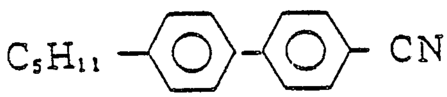

35 wt. %

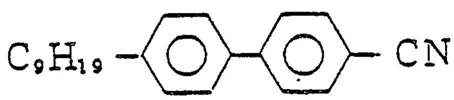

25 wt. %

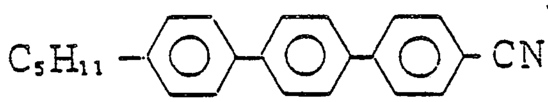

20 wt. %

was filled in a cell provided with transparent electrodes each obtained by coating polyvinyl alcohol (PVA) as an aligning agent and rubbing the resulting surface to subject it to a parallel aligning treatment and having a distance between the electrodes of 10 µm to prepare a TN mode display cell, which was then observed under a polarizing microscope. As a result, a reverse domain was observed to be formed.

To this nematic liquid crystal composition was added a compound of the present invention, i.e.

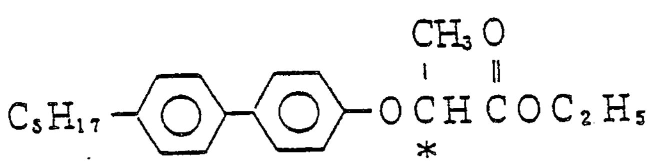

in 0.1% by weight, followed by observing a TN mode cell similarly prepared. As a result, the reverse domain was dissolved and a uniform nematic phase was observed.

## Claims

1. Optically active compounds of the formula:

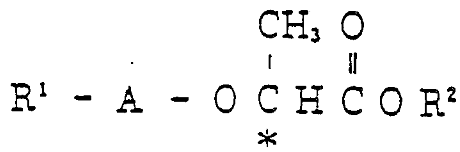

(I)

in which $R^1$ is a straight or branched alkyl or alkoxy group containing up to 18 carbon atoms, $R^2$ is a straight or branched alkyl group containing up to 12 carbon atoms, -A- is a group

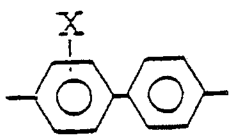 , 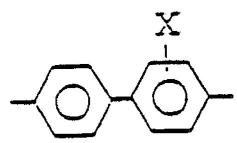 , 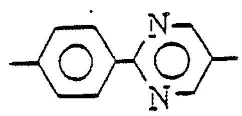 , 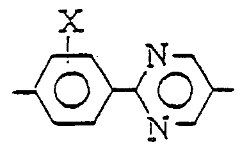 ,

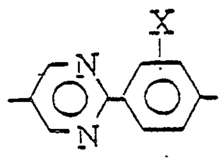 , 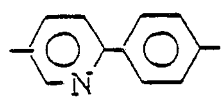 or 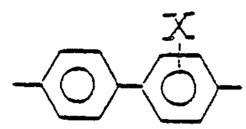

(wherein X is a fluorine or chlorine atom or a cyano group and * indicates an asymmetric carbon atom.

2. Compounds as claimed in claim 1 in which $R^1$ is a straight or branched alkyl or alkoxy group containing from 4 to 15 carbon atoms.

3. Compounds as claimed in claim 2 in which $R^1$ is a straight or branched a alkyl or alkoxy group containing from 6 to 12 carbon atoms.

4. Compounds as claimed in any one of the preceding claims in which $R^2$ is a straight or branched alkyl group containing from 2 to 10 carbon atoms.

5. Compounds as claimed in any one of the preceding claims in which A is a group:

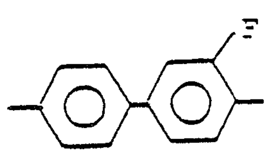 , 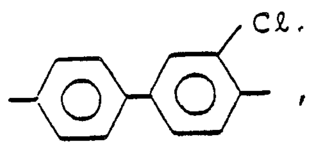 , 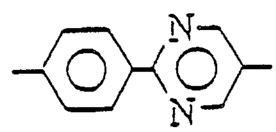 ,

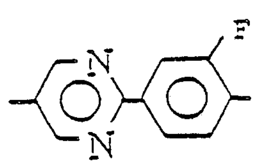 , 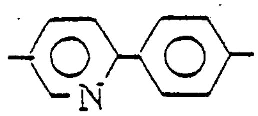 or 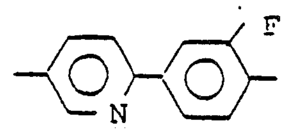

6. A liquid crystal composition comprising at least two components at least one of which is an optically active compound as claimed in claim 1.

7. A liquid crystal composition according to claim 6, exhibiting a cholesteric liquid crystal phase.

8. A liquid crystal composition according to claim 6, exhibiting a chiral smectic phase.

9. A light switching element comprising a liquid crystal composition as claimed in claim 6.